# EUROPÄISCHE PATENTSCHRIFT

(11) **EP 2 879 725 B1**
(45) Veröffentlichungstag und Bekanntmachung des Hinweises auf die Patenterteilung: **16.09.2020**
(21) Anmeldenummer: 13744487.3
(22) Anmeldetag: 26.07.2013
(51) Int. Cl.: A61L 27/10, A61L 27/30, A61L 27/44, A61L 27/56

(54) **MEHRKOMPONENTEN-FÜGEN VON PLASTISCHEN ZUBEREITUNGEN ZUR HERSTELLUNG VON MEDIZINPRODUKTEN MIT FUNKTIONELLER OBERFLÄCHE**
MULTI-COMPONENT JOINING OF PLASTIC PREPARATIONS IN ORDER TO PRODUCE MEDICAL PRODUCTS WITH FUNCTIONAL SURFACES
ASSEMBLAGE À PLUSIEURS COMPOSANTS DE PRÉPARATIONS PLASTIQUES POUR LA PRODUCTION DE PRODUITS MÉDICAUX COMPRENANT UNE SURFACE FONCTIONNELLE

(30) Priorität: 30.07.2012 DE 102012213348
(43) Veröffentlichungstag der Anmeldung: 10.06.2015
(73) Patentinhaber: CeramTec GmbH, 73207 Plochingen (DE)
(72) Erfinder: KUNTZ, Meinhard, 73733 Esslingen (DE); MESSMER, Moritz, 70372 Stuttgart (DE); HEINZMANN, Simon, 73312 Geislingen/Aufhausen (DE)
(74) Vertreter: Fehrenbacher, Eckhard Anton
(86) Internationale Anmeldenummer: PCT/EP2013/065810
(87) Internationale Veröffentlichungsnummer: WO 2014/019954

(56) Entgegenhaltungen:
- DE-A1- 3 832 942
- DE-A1- 19 945 529
- DE-A1-102007 020 471
- DE-A1-102008 001 402
- US-A1- 2010 076 570
- US-B1- 6 302 913
- TRAINI T ET AL: "Direct laser metal sintering as a new approach to fabrication of an isoelastic functionally graded material for manufacture of porous titanium dental implants", DENTAL MATERIALS, ELSEVIER, AMSTERDAM, NL, vol. 24, no. 11, 1 November 2008 (2008-11-01), pages 1525-1533, XP025434248, ISSN: 0109-5641, DOI: 10.1016/J.DENTAL.2008.03.029 [retrieved on 2008-05-27]

## Beschreibung

Die Erfindung betrifft ein Verfahren zur Herstellung von Medizinprodukten mit funktionellen Oberflächen. Insbesondere betrifft die Erfindung ein Verfahren mit dem keramische Implantate mit knochenaffinen Oberflächen hergestellt werden können sowie solcher Art hergestellte Medizinprodukte.

Strukturkeramische Komponenten, insbesondere Implantate, Prothesen oder Ähnliches werden heute oft mit einer funktionellen Beschichtung, beispielsweise einer knochenaffinen oder antiseptischen Beschichtung oder Schicht versehen. Die funktionelle Oberfläche soll die Einbindung der Komponente in den Körper verbessern. Insbesondere geht es hier häufig um ein schnelleres und haltbareres Einwachsen der Komponente, was beispielsweise durch poröse Beschichtungen oder Oberflächen erreicht werden kann. Die auf der Oberfläche der Komponente vorhandenen Poren können das Einwachsen von Knochenbestandteilen ermöglichen und fördern, so dass ein sicherer Halt eines Implantats durch körpereigene Einbindung möglich wird. Andererseits müssen häufig auch entzündliche Prozesse beherrscht werden, die beim Einsetzen eines Implantats in den Körper oft unvermeidbar sind. Komponenten, die als Gelenkersatz verwendet werden, benötigen grundsätzlich eine Funktionsschicht zur Verbindung mit dem Knochen.

Solche Implantate, insbesondere auch aus Keramik, sind aus dem Stand der Technik bekannt. Sie werden im Allgemeinen in mehreren Arbeitsgängen hergestellt, wobei auf den Grundkörper, der massiv und tragend ist, eine funktionelle oder poröse Oberfläche in irgendeiner Art und Weise, beispielsweise durch Beschichtung aufgebracht wird. Diese Herstellungsverfahren sind vergleichsweise zeitaufwändig und umständlich, weil verschiedene Verfahren für die Formung des Grundkörpers und die anschließende Oberflächenbehandlung benötigt werden.

So zeigt DE38 32 942 A1 einen keramischen Verbundkörper mit einem dichten und einem porösen Teil und dessen Herstellung. Der dichte und der poröse Teil werden hier in verschiedenen Arbeitsgängen erzeugt und im Anschluss zusammengebracht und gesintert. Der poröse Teil weist dazu eine Bohrung oder Aussparung auf, in die ein Teil des dichten Keramikteils eingebracht wird. Während des Sinterns wird das dichte Keramikteil aufgrund des höheren Schrumpfungsfaktors des porösen Keramikteils in der Bohrung oder Aussparung fest zusammengedrückt, wodurch sich der Verbundkörper ergibt.

Die Aufgabe der Erfindung ist daher das Bereitstellen eines Verfahrens, das die Herstellung eines Implantats, beispielsweise einer Prothese, mit einem Kernmaterial und einem funktionellen Oberflächenmaterial vereinfacht.

Die Aufgabe wird durch ein Verfahren gemäß Anspruch 1 gelöst; vorteilhafte Ausgestaltungen dieses Verfahrens sind in den Unteransprüchen definiert.

Demgemäß sieht ein erfindungsgemäßes Verfahren zur Herstellung eines Implantats mit einer funktionellen Oberfläche vor, dass ein Grundkörper und die funktionelle Oberfläche in einem Arbeitsgang hergestellt werden, wobei die Erfindung keramische Komponenten mit einem massiven, lasttragenden Bereich und mit einer porösen, knochenaffinen Grenzschicht oder Oberfläche vorsieht.

Für die herzustellende Hochleistungskeramik wird eine entsprechende Pulvermischung aufbereitet, bei der sowohl die Homogenisierung der einzelnen Zuschlagstoffe als auch die Dispergierung der Pulveragglomerate gemäß Stand der Technik realisiert wird. Beispielsweise kommen hier Zirkonoxid, Siliziumnitrid, Aluminiumoxid oder auch Verbundwerkstoffe wie ZTA und/oder Mischungen der genannten Stoffe in Frage. Außerdem können Edukte verwendet werden, die zu den genannten Stoffen, beispielsweise durch Sintern gegebenenfalls unter einer bestimmten Atmosphäre, umgesetzt werden können. Die Pulvermischung wird mit einem plastischen Bindersystem versetzt und bildet so eine Formmasse (Feedstock), mit der eine Formgebung über Hoch- oder Niederdruckspritzguss oder auch über Extrusion möglich ist.

Ein Teil des Feedstocks kann mit nicht-plastischen, partikelförmigen Zuschlagstoffen (Platzhalter) versetzt werden, die nach der Formgebung, beispielsweise beim anschließenden Sinterprozess, weitgehend rückstandsfrei wieder entfernt werden und dabei Poren hinterlassen. Als Zuschlagstoffe können beispielsweise Polyethylen, Polystyrol oder ähnliche organische Kohlenstoffverbindungen oder auch Graphit zugegeben werden. Gemäß einer bevorzugten Ausführungsform der Erfindung werden die Platzhalter so in den Feedstock eingebracht, dass nach der Sinterung in dem Körper Poren verbleiben, die für das Einwachsverhalten in den Knochen besonders vorteilhaft sind.

Ein besonders bevorzugtes Verfahren zur Herstellung von Implantaten mit zumindest einer funktionellen Oberfläche umfasst die folgenden Schritte:
a) Aufbereitung einer keramischen Pulvermischung,
b) Versetzen der keramischen Pulvermischung mit einem plastischen Bindersystem, wobei das plastische Bindersystem an ein sich anschließendes Formgebungsverfahren angepasst ist, und die keramische Pulvermischung mit dem plastischen Bindersystem einen ersten Feedstock bildet,
c1) Teilen des ersten Feedstocks und Versetzen eines Teils des Feedstocks mit Zuschlagstoffen zur Bildung eines zweiten Feedstocks, oder
c2) Herstellen eines zweiten Feedstocks nach den Schritten a) und b),
d) Durchführen eines Formgebungsverfahrens, bei dem der Grundkörper und die knochenaffine Oberfläche aus dem ersten und dem zweiten Feedstock geformt werden,
e) Entbindern des Grünkörpers,
f) Sintern des im vorherigen Schritt geformten und entbinderten Braunlings des Implantats zum fertigen Implantat mit einer funktionellen und insbesondere knochenaffinen Oberfläche.

Gemäß einer besonders bevorzugten Ausführungsform der Erfindung können erste und zweite Feedstocks sowohl mit als auch ohne Platzhalter/Zuschlagstoffe in folgender Weise hergestellt und verarbeitet werden:
- Die Feedstocks mit und ohne Platzhalter können über Spritzguss und/oder Extrudieren jeweils separat zur Formgebung verwendet werden.
- Beim Formgebungsprozess können die Feedstocks unter kontrollierten Bedingungen miteinander in Verbindung gebracht werden. Dabei ermöglicht das ausgewählte organische Bindersystem eine Haftverbindung der plastischen Massen. Der Formgebungsprozess ist typischerweise thermisch aktiviert mit Temperaturen zwischen 80°C - 170°C.
- Die Feedstocks sind so eingestellt, dass sie unter gleichen Bedingungen gesintert werden können und dabei eine so ähnliche Sinterkinetik aufweisen, dass die Verbindung des massiven Feedstocks mit dem Platzhalter-haltigen Feedstock bei der Sinterung erhalten bleibt und dabei durch die Sinterung eine Festkörperverbindung entsteht.
- Die beiden Feedstocks werden durch Zweikomponenten-Spritzguss oder Zweikomponenten-Extrusion verarbeitet und dabei in die gewünschte Form gebracht. Erfindungsgemäß werden durch die Mehrkomponenten-Formgebung die späteren massiven, lasttragenden Bereiche des Bauteils mit dem massiven Feedstock und die porösen, knochenaffinen Bereiche mit dem Zuschlagstoff-/Platzhalter-haltigen Feedstock gefüllt. Dadurch sind im Rahmen der Grenzen dieser beiden Verfahren beliebige Formkörper herstellbar, mit denen das Ziel massiver Festkörper mit knochenaffiner poröser Oberfläche praktisch beliebig realisiert werden kann. Je nach Anwendung, beispielsweise bei Spacern für Wirbelsäulenimplantate, kann der poröse Bereich auch größere Bereiche oder durchgängige Kanäle des Bauteils einnehmen.
- Nach der thermisch aktivierten Formgebung entsteht nach Abkühlung ein relativ fester "Grünkörper", der das Keramikpulver, das organische Plastifizierungsmittel und die Platzhalter enthält. Die Plastifizierungsmittel werden dann entfernt, beispielsweise durch Verdampfen oder durch Säurewaschen. Der entbinderte Grünkörper (Braunling) wird gesintert, wobei erfindungsgemäß die gewünschten massiven und porösen Bereiche entstehen. Die Platzhalter werden ausgebrannt. Der Übergangsbereich zwischen massiver und poröser Keramik wird durch den Sinterprozess verfestigt.

Die vorliegende Erfindung wird anhand der nachfolgenden bevorzugten Ausführungsformen beschrieben. Die vorliegende Erfindung betrifft in der ersten Ausführungsform ein Verfahren zur Herstellung eines Implantats mit mindestens einer funktionellen Oberfläche, wobei ein massiver Grundkörper und die funktionelle Oberfläche in einem Arbeitsgang hergestellt werden und die funktionelle Oberfläche eine knochenaffine Oberfläche ist und als Basiswerkstoff ein keramisches Pulver verwendet wird.
Ausführungsform 2: Verfahren nach der vorstehenden Ausführungsform, wobei das keramische Pulver Zirkonoxid, Siliziumnitrid, Aluminiumoxid und/oder Verbundwerkstoffe wie ZTA umfasst und/oder zu diesen Stoffen umsetzbar ist.
Ausführungsform 3: Verfahren nach einer der vorstehenden Ausführungsformen, wobei der Grundkörper und das Material der knochenaffinen Oberfläche aus demselben Basiswerkstoff gefertigt werden.
Ausführungsform 4: Verfahren nach einer der vorstehenden Ausführungsformen, dadurch gekennzeichnet, dass der Grundkörper und das Material der knochenaffinen Oberfläche ein plastisches Bindersystem umfasst, das eine Haftverbindung zwischen dem Grundkörper und dem Material der knochenaffinen Oberfläche nach der Aushärtung ermöglicht, wobei es sich bei dem plastischen Bindersystem bevorzugt um ein organisches Bindersystem handelt.
Ausführungsform 5: Verfahren nach einer der vorstehenden Ausführungsformen, wobei einem Teil des Basiswerkstoffs, der als Grundlage für das Material der knochenaffinen Oberfläche dient, Zuschlagstoffe zugegeben werden, die nach der Formgebung, insbesondere durch Ausbrennen, wieder entfernt werden, wodurch eine definierte Porosität der knochenaffinen Oberfläche eingestellt wird.
Ausführungsform 6: Verfahren nach der vorstehenden Ausführungsformen, wobei die Zuschlagstoffe partikelförmig sind und bevorzugt Polyethylen, Polystyrol, Graphit und/oder organische Kohlenstoffverbindungen und/oder Mischungen aus diesen Stoffen umfassen.
Ausführungsform 7: Verfahren nach einer der Ausführungsformen 1 bis 6, umfassend die folgenden Schritte:
   a) Aufbereitung einer keramischen Pulvermischung,
   b) Versetzen der keramischen Pulvermischung mit einem plastischen Bindersystem, wobei das plastische Bindersystem an ein sich anschließendes Formgebungsverfahren angepasst ist, und die keramische Pulvermischung mit dem plastischen Bindersystem einen ersten Feedstock bildet,
   c1) Teilen des ersten Feedstocks und Versetzen eines Teils des Feedstocks mit Zuschlagstoffen zur Bildung eines zweiten Feedstocks, oder
   c2) Herstellen eines zweiten Feedstocks nach den Schritten a) und b),
   d) Durchführen eines Formgebungsverfahrens, bei dem der Grundkörper und die knochenaffine Oberfläche aus dem ersten und dem zweiten Feedstock geformt werden,
   e) Entbindern des Grünlings,
   f) Sintern des im vorherigen Schritt geformten und entbinderten Braunlings des Implantats zum fertigen Implantat mit einer knochenaffinen Oberfläche.
Ausführungsform 8: Verfahren nach Ausführungsform 7, wobei der Grundkörper und/oder die knochenaffine Oberfläche durch Hoch- oder Niederdruckspritzguss oder durch Extrusion, insbesondere durch Zweikomponenten-Spritzguss oder Zweikomponenten-Extrusion geformt werden.
Ausführungsform 9: Verfahren nach Ausführungsform 8: wobei der Formgebungsprozess thermisch aktiviert wird, bevorzugt bei Temperaturen zwischen 80°C - 170°.
Ausführungsform 10: Verfahren nach einer der Ausführungsformen 7 bis 9, wobei die Feedstocks so zusammengesetzt sind, dass sie unter den gleichen Bedingungen gesintert werden können und dabei eine so ähnliche Sinterkinetik aufweisen, dass eine feste Verbindung zwischen dem Grundkörper und der knochenaffinen Oberfläche entsteht.
Ausführungsform 11: Implantat, umfassend einen Grundkörper und zumindest eine funktionelle Oberfläche, herstellbar nach einem Verfahren gemäß der Ausführungsformen 1 bis 10:

## Patentansprüche

1. Verfahren zur Herstellung eines Implantats mit mindestens einer funktionellen Oberfläche, **dadurch gekennzeichnet, dass** ein massiver Grundkörper und die funktionelle Oberfläche in einem Arbeitsgang hergestellt werden, wobei die funktionelle Oberfläche eine poröse, knochenaffine Oberfläche ist, und als Basiswerkstoff ein keramisches Pulver verwendet wird.

2. Verfahren nach Anspruch 1, **dadurch gekennzeichnet, dass** das keramische Pulver Zirkonoxid, Siliziumnitrid, Aluminiumoxid und/oder Verbundwerkstoffe wie ZTA umfasst und/oder zu diesen Stoffen umsetzbar ist.

3. Verfahren nach einem der vorstehenden Ansprüche, **dadurch gekennzeichnet, dass** der Grundkörper und das Material der knochenaffinen Oberfläche aus demselben Basiswerkstoff gefertigt werden.

4. Verfahren nach einem der vorstehenden Ansprüche, **dadurch gekennzeichnet, dass** der Grundkörper und das Material der knochenaffinen Oberfläche ein plastisches Bindersystem umfasst, das eine Haftverbindung zwischen dem Grundkörper und dem Material der knochenaffinen Oberfläche nach der Aushärtung ermöglicht, wobei es sich bei dem plastischen Bindersystem um ein organisches Bindersystem handelt.

5. Verfahren nach einem der vorstehenden Ansprüche, **dadurch gekennzeichnet, dass** einem Teil des Basiswerkstoffs, der als Grundlage für das Material der knochenaffinen Oberfläche dient, Zuschlagstoffe zugegeben werden, die nach der Formgebung wieder entfernt werden, wodurch eine definierte Porosität der knochenaffinen Oberfläche eingestellt wird.

6. Verfahren nach dem vorstehenden Anspruch, **dadurch gekennzeichnet, dass** die Zuschlagstoffe partikelförmig sind und Polyethylen, Polystyrol, Graphit und/oder organische Kohlenstoffverbindungen und/oder Mischungen aus diesen Stoffen umfassen.

7. Verfahren nach einem der Ansprüche 1 bis 6, umfassend die folgenden Schritte:
a) Aufbereitung einer keramischen Pulvermischung,
b) Versetzen der keramischen Pulvermischung mit einem plastischen Bindersystem, wobei das plastische Bindersystem an ein sich anschließendes Formgebungsverfahren angepasst ist, und die keramische Pulvermischung mit dem plastischen Bindersystem einen ersten Feedstock bildet,
c1) Teilen des ersten Feedstocks und Versetzen eines Teils des Feedstocks mit Zuschlagstoffen zur Bildung eines zweiten Feedstocks, oder
c2) Herstellen eines zweiten Feedstocks nach den Schritten a) und b),
d) Durchführen eines Formgebungsverfahrens, bei dem der Grundkörper und die knochenaffine Oberfläche aus dem ersten und dem zweiten Feedstock geformt werden,
e) Entbindern des Grünlings,
f) Sintern des im vorherigen Schritt geformten und entbinderten Braunlings des Implantats zum fertigen Implantat mit einer knochenaffinen Oberfläche.

8. Verfahren nach Anspruch 7, **dadurch gekennzeichnet, dass** der Grundkörper und/oder die knochenaffine Oberfläche durch Hoch- oder Niederdruckspritzguss oder durch Extrusion, insbesondere durch Zweikomponenten-Spritzguss oder Zweikomponenten-Extrusion geformt werden.

9. Verfahren nach Anspruch 8, **dadurch gekennzeichnet, dass** der Formgebungsprozess thermisch aktiviert wird.

10. Verfahren nach einem der Ansprüche 7 bis 9, **dadurch gekennzeichnet, dass** die Feedstocks so zusammengesetzt sind, dass sie unter den gleichen Bedingungen gesintert werden können und dabei eine so ähnliche Sinterkinetik aufweisen, dass eine feste Verbindung zwischen dem Grundkörper und der knochenaffinen Oberfläche entsteht.

11. Implantat, umfassend einen Grundkörper und zumindest eine funktionelle Oberfläche, herstellbar nach einem Verfahren gemäß einem der vorstehenden Ansprüche.

## Claims

1. Method for producing an implant having at least one functional surface, **characterized in that** a solid base body and the functional surface are produced in one work step, the functional surface being a porous, bone-affine surface, and a ceramic powder being used as the base material.

2. Method according to claim 1, **characterized in that** the ceramic powder comprises zirconium oxide, silicon nitride, aluminum oxide and/or composite materials such as ZTA and/or can be converted into these substances.

3. Method according to either of the preceding claims, **characterized in that** the base body and the material of the bone-affine surface are made from the same base material.

4. Method according to any of the preceding claims, **characterized in that** the base body and the material of the bone-affine surface comprise a plastic binder system which allows the base body and the material of the bone-affine surface to be adhesively bonded after curing, the plastic binder system being an organic binder system.

5. Method according to any of the preceding claims, **characterized in that** additives are added to a part of the base material that serves as the basis for the material of the bone-affine surface, which additives are removed again after shaping, as a result of which a defined porosity of the bone-affine surface is set.

6. Method according to the preceding claim, **characterized in that** the additives are particulate and comprise polyethylene, polystyrene, graphite and/or organic carbon compounds and/or mixtures of these substances.

7. Method according to any of claims 1 to 6, comprising the following steps:
a) preparing a ceramic powder mixture;
b) mixing the ceramic powder mixture with a plastic binder system, wherein the plastic binder system is adapted to a subsequent shaping method, and the ceramic powder mixture forms, together with the plastic binder system, a first feedstock;
c1) dividing the first feedstock and mixing part of the feedstock with additives to form a second feedstock; or
c2) producing a second feedstock according to steps a) and b);
d) carrying out a shaping method in which the base body and the bone-affine surface are formed from the first and the second feedstock;
e) debinding the green body;
f) sintering the brown body of the implant that was formed and debound in the previous step to produce the finished implant having a bone-affine surface.

8. Method according to claim 7, **characterized in that** the base body and/or the bone-affine surface are formed by high- or low-pressure injection molding or by extrusion, in particular by two-component injection molding or two-component extrusion.

9. Method according to claim 8, **characterized in that** the shaping process is thermally activated.

10. Method according to any of claims 7 to 9, **characterized in that** the feedstocks are composed such that they can be sintered under the same conditions and have such a similar sintering kinetics that a stable bond is produced between the base body and the bone-affine surface.

11. Implant comprising a base body and at least one functional surface, producible by a method according to any of the preceding claims.

## Revendications

1. Procédé de production d'un implant comportant au moins une surface fonctionnelle, **caractérisé en ce qu'**un corps principal compact et la surface fonctionnelle sont produits en une seule opération, la surface fonctionnelle étant une surface poreuse ayant des affinités avec les os, et une poudre céramique étant utilisée comme matériau de base.

2. Procédé selon la revendication 1, **caractérisé en ce que** la poudre céramique comprend de l'oxyde de zirconium, du nitrure de silicium, de l'oxyde d'aluminium et/ou des matériaux composites tels que le ZTA, et/ou **en ce qu'**elle peut être transformée en ces substances.

3. Procédé selon l'une des revendications précédentes, **caractérisé en ce que** le corps principal et le matériau de la surface ayant des affinités avec les os sont réalisés à partir du même matériau de base.

4. Procédé selon l'une des revendications précédentes, **caractérisé en ce que** le corps principal et le matériau de la surface ayant des affinités avec les os comprennent un système de liant plastique, lequel permet, après le durcissement, une liaison adhésive entre le corps principal et le matériau de la surface ayant des affinités avec les os, le système de liant plastique étant un système de liant organique.

5. Procédé selon l'une des revendications précédentes, **caractérisé en ce que** des additifs sont ajoutés à une partie du matériau de base servant de base au matériau de la surface ayant des affinités avec les os, lesquels additifs sont éliminés après la mise en forme, ce qui permet de régler une porosité définie de la surface ayant des affinités avec les os.

6. Procédé selon la revendication précédente, **caractérisé en ce que** les additifs sont particulaires et comprennent du polyéthylène, du polystyrène, du graphite et/ou des composés carbonés organiques et/ou des mélanges de ces substances.

7. Procédé selon l'une des revendications 1 à 6, comprenant les étapes suivantes :
a) préparation d'un mélange pulvérulent céramique,
b) mélange du mélange pulvérulent céramique avec un système de liant plastique, le système de liant plastique étant adapté à un procédé ultérieur de mise en forme, et le mélange pulvérulent céramique formant une première charge avec le système de liant plastique,
c1) division de la première charge et mélange d'une partie de la charge avec des additifs pour former une seconde charge, ou
c2) production d'une seconde charge selon les étapes a) et b),
d) réalisation d'un procédé de mise en forme dans lequel le corps principal et la surface ayant des affinités avec les os sont formés à partir des première et seconde charges,
e) déliantage du corps cru,
f) frittage de la pièce brune de l'implant qui est formée et déliantée à l'étape précédente pour obtenir l'implant réalisé comportant une surface ayant des affinités avec les os.

8. Procédé selon la revendication 7, **caractérisé en ce que** le corps principal et/ou la surface ayant des affinités avec les os sont formés par moulage par injection haute ou basse pression ou par extrusion, en particulier par moulage par injection à deux composants ou extrusion à deux composants.

9. Procédé selon la revendication 8, **caractérisé en ce que** le processus de mise en forme est activé thermiquement.

10. Procédé selon l'une des revendications 7 à 9, **caractérisé en ce que** les charges sont composées de façon à pouvoir être frittées dans les mêmes conditions et présentent ainsi une cinétique de frittage similaire de façon à former une liaison ferme entre le corps principal et la surface ayant des affinités avec les os.

11. Implant comprenant un corps principal et au moins une surface fonctionnelle, lequel peut être produit par un procédé selon l'une des revendications précédentes.
